Europäisches Patentamt

(19) European Patent Office

Office européen des brevets

(11) Publication number: **0 153 018**
B1

# (12) EUROPEAN PATENT SPECIFICATION

(45) Date of publication of the patent specification:
16.09.87

(51) Int. Cl.⁴: **C 07 C 101/24,** C 07 C 149/20, A 23 K 1/16

(21) Application number: 85300388.7

(22) Date of filing: 21.01.85

(54) Lysine salt crystals and process for produktion thereof.

(30) Priority: 13.02.84 JP 24876/84

(43) Date of publication of application:
28.08.85 Bulletin 85/35

(45) Publication of the grant of the patent:
16.09.87 Bulletin 87/38

(84) Designated Contracting States:
DE FR GB NL

(56) References cited:
BE - A - 887 922

CHEMICAL ABSTRACTS, vol. 98, no. 23, June 6, 1983,
page 531, abstract no. 196869f, Columbus, Ohio, US;
L.E. RUSSELL et al.: "Tryptophan, threonine, isoleucine
and methionine supplementation of a 12% protein,
lysine-supplemented, corn-soybean meal diet for
growing pigs"

The file contains technical information submitted after
the application was filed and not included in this
specification

(73) Proprietor: AJINOMOTO CO., INC., 5-8,
Kyobashi 1-chome, Chuo-ku, Tokyo 104 (JP)

(72) Inventor: Tanaka, Kiyoshi, 1-16-2, Ueno, Taito-ku Tokyo
(JP)
Inventor: Koga, Yoshihiro, 373-7, Oazahonjyo
Honjyo-machi, Saga-hi Saga-ken (JP)
Inventor: Saeki, Masaru, 6226, Tsujido, Fujisawa-shi
Kanagawa-ken (JP)
Inventor: Kaneko, Tetsuya, 2-20-8, Kannon Kawasaki-ku,
Kawasaki-shi Kanagawa-ken (JP)
Inventor: Kawakita, Tetsuya, 3-31-23, Mutsuura-cho
Kanazawa-ku, Yokohama-shi Kanagawa-ken (JP)

(74) Representative: Harrison, David Christopher et al,
MEWBURN ELLIS & CO 2/3 Cursitor Street, London
EC4A 1BQ (GB)

## Description

This invention relates to novel lysine salt crystals and to a process for the production thereof.

Lysine hydrochloride is a widely used chemical, for example, as an animal feed additive. It adsorbs moisture and may undergo aggregation. This detracts from its convenience in use and its commercial value.

2-Hydroxy-4-methylthiobutyric acid, also called methionine hydroxy analog, is a compound having a hydroxyl group in the place of the amino group of methionine. It is also known as an intermediate for the synthesis of methionine. Since it is easily converted into methionine within the animal body, it is utilized in animal feed instead of methionine. However this compound cannot be easily produced in the form of crystals. It is usually marketed in the form of a concentrated aqueous solution. Having a strong acidity which may even be below pH 1.0, this concentrated aqueous solution poses handling problems.

The present invention concerns itself with the elimination of both these disadvantages; it is based on the discovery of equimolar salt crystals of lysine and 2-hydroxy-4-methylthiobutyric acid which adsorb moisture minimally and have a low tendency to aggregation.

Specifically, this invention relates to equimolar salt crystals of lysine and 2-hydroxy-4-methylthiobutyric acid and to a process for the production thereof.

In CA 98:196869F it is disclosed that a lysine-enriched animal feedstuff may include methionine hydroxy analog and various further supplements. From the original document [J. Animal Sci 1983 56(5)1115-23] it is clear that the lysine was present as lysine hydrochloride. In BE-A-887922 there is description of the presence of the preparation of equimolecular salts of lysine and carboxy methyl cystine, which are stated to be useful in various specific pharmaceutical applications concerned with the bronchial tract.

The lysine-2-hydroxy-4-methylthiobutyric acid salt crystals provided by this invention are better than lysine hydrochloride in terms of freedom from hygroscopicity and are better than the usual 2-hydroxy-4-methylthiobutyric acid in terms of ease of handling. They will be very useful as an animal feed additive which is itself a source for both methionine and lysine.

The production of lysine-2-hydroxy-4-methylthiobutyric acid equimolar salt crystals is effected by causing lysine and 2-hydroxy-4-methylthiobutyric acid to react with each other in an aqueous medium and subsequently concentrating, crystallizing, and separating the resultant reaction mixture.

Lysine may be in either of the two forms, L-form or DL-form. It can be advantageously used in an equimolar or in a slight excess amount relative to the 2-hydroxy-4-methylthiobutyric acid. The equimolar salt is obtained even when 2-hydroxy-4-methylthiobutyric acid is used in an amount twice the molarity of lysine.

The present invention will be described more specifically below with reference to the following Examples.

*Example 1*

To 100 g of 2-hydroxy-4-methylthiobutyric acid (hereinafter referred to as «MHA» for short) (aqueous 88% MHA solution produced by Monsanto Company and marketed under trademark designation of «ALIMET»), 67.8 g of L-Lysine was added. These amounts correspond to a MHA : Lysine molar ratio of 1.3 : 1. The resultant solution was concentrated to 1.5 times the original level, then cooled from 60°C to 20°C, and subjected to crystallization and separation, to obtain 64 g of crude crystals. The crude crystals were dissolved in 300 ml of water. The solution and 4 g of activated carbon added thereto were stirred at 60°C for one hour and then filtered. The filtrate was concentrated to 10 times the original level, cooled from 60°C to 20°C, and subjected to crystallization, to afford 6.0 g of 1: 1 mol salt of L-lysine-2-hydroxy-4-methylthiobutiric acid.

The physical properties of the product were as follows.

(1) Elementary analysis:

| For $C_{11}H_{24}O_5N_2S$ | | Calculated | Found |
|---|---|---|---|
| | C | 44.57% | 44.44% |
| | H | 8.18 | 8.33 |
| | N | 9.45 | 9.54 |
| | O | 26.99 | 26.98 |
| | S | 10.82 | 10.70 |

(2) Molecular weight: 296.43 (identified to be $C_{11}H_{24}O_5N_2S$ by elementary analysis)

(3) Melting point: 169.5°C

(4) Solubility in solvents: Readily soluble in water and sparingly soluble in methanol and ethanol.

(5) Crystal form: Needle.

*Example 2*

By repeating the procedure of Example 1 using 170 g of «ALIMET» and 146 g of L-lysine (molar ratio MHA : L-lysine = 1 : 1), 53 g of crude crystals were obtained. By refining the crude crystals by the procedure of Example 1, there were obtained 4.3 g of 1 : 1 mol salt crystals of L-lysine-2-hydroxy-4-methylthiobutyric acid.

*Example 3*

By repeating the procedure of Example 1 using 170 g of «ALIMET» and 73 g of L-lysine (molar ratio MHA : L-lysine = 2 : 1), 23 g of crude ctystals were obtained. By refining the crude crystals by the procedure of Example 1, there were obtained 3.0 g of 1 : 1 mol salt crystals of L-lysine-2-hydroxy-4-methylthiobutyric acid.

*Comparative Example*

In desiccators kept under relative humidity of 54%, (a) 3.0 g of 1 : 1 mol salt crystals of L-lysine-2-hydroxy-4-methylthiobutyric acid and separately (b) 3.0 g of L-lysine hydrochloride were left standing for 20 days. At the end of the period they were tested for moisture gain. The same test was repeated under relative humidity of 66%. The results are shown below.

The samples used had initial water content of 0.2%.

|  | RH 54% | RH 66% |
|---|---|---|
| Lysine hydrochloride | 3.0% | 7.5% |
| Lysine-2-hydroxy-4-methylthiobutyric acid salt | 1.8% | 1.8% |

It is noted from the foregoing table that the novel lysine salt of this invention has extremely low hygroscopicity as compared with lysine hydrochloride.

## Claims

1. Equimolar salt crystals of lysine and 2-hydroxy-4-methylthiobutiric acid.

2. The crystals as claimed in claim 1, for use in animal feedstuffs.

3. A process for the production of equimolar salt crystals of lysine and 2-hydroxy-4-methylthiobutyric acid, wherein lysine and 2-hydroxy-4-methylthiobutyric acid are reacted with each other in aqueous solution, with subsequent crystallizing and separating of the resultant reaction product.

4. A process according to claim 3 wherein the molar ratio lysine : 2-hydroxy-4-methylthiobutyric acid in the solution is between 1 : 1 and 1 : 3.

5. A process acording to claim 3 or claim 4, wherein the separation includes a recrystallization of the reaction product.

## Patentansprüche

1. Kristalle des äquimolaren Salzes von Lysin und 2-Hydroxy-4-methylthiobuttersäure.

2. Kristalle gemäss Anspruch 1 zur Verwendung in Tierfuttermitteln.

3. Verfahren zur Herstellung von Kristallen des äquimolaren Salzes von Lysin und 2-Hydroxy-4-methylthio-buttersäure, bei dem Lysin und 2-Hydroxy-4-methylthiobuttersäure in wässriger Lösung miteinander umgesetzt werden und nachfolgend das gebildete Reaktionsprodukt kristallisiert und abgetrennt wird.

4. Verfahren gemäss Anspruch 3, bei dem das Molverhältnis Lysin : 2-Hydroxy-4-methylthiobuttersäure in der Lösung zwischen 1 : 1 und 1 : 3 ist.

5. Verfahren gemäss Anspruch 3 oder Anspruch 4, bei dem die Abtrennung die Umkristallisation des Reaktionsprodukts einschliesst.

## Revendications

1. Sel équimolaire cristallisé de lysine et d'acide 2-hydroxy-4-méthylthiobutyrique.

2. Le sel cristallisé selon la revendication 1, pour l'utilisation dans les aliments pour animaux.

3. Un procédé pour la fabrication d'un sel équimolaire cristallisé de lysine et d'acide 2-hydroxy-4-méthylthiobutyrique, dans lequel on fait réagir la lysine et l'acide 2-hydroxy-4-méthylthiobutyrique l'une avec l'autre en solution aqueuse, avec cristallisation et séparation subséquentes du produit de réaction résultant.

4. Un procédé selon la revendication 3, dans lequel le rapport molaire lysine : acide 2-hydroxy-4-méthylthiobutyrique dans la solution est compris entre 1 : 1 et 1 : 3.

5. Un procédé selon la revendication 3 ou 4, dans lequel la séparation comprend une recristallisation du produit de réaction.